# EUROPEAN PATENT SPECIFICATION

(11) **EP 1 629 769 B1**
(45) Date of publication and mention of the grant of the patent: **11.02.2009**
(21) Application number: 05018415.9
(22) Date of filing: 24.08.2005
(51) Int. Cl.: A61B 5/0404

(54) **Portable electrocardiograph and processing method**
Tragbarer Elektrokardiograph sowie Verfahren zur Anwendung
Electrocardiographe portable et méthode de traitement des signaux ECG associés.

(30) Priority: 27.08.2004 JP 2004248807
(43) Date of publication of application: 01.03.2006
(73) Proprietor: Omron Healthcare Co., Ltd., Kyoto 615-0084 (JP)
(72) Inventor: Moroki, Yoko c/o Omron Healthcare Co., Ltd, Kyoto-shi Kyoto 615-0084 (JP); Yamamoto, Norihito c/o Omron Healthcare Co., Ltd, Kyoto-shi Kyoto 615-0084 (JP); Ishida, Junichi c/o Omron Healthcare Co., Ltd, Kyoto-shi Kyoto 615-0084 (JP); Tanabe, Kazuhisa c/o Omron Healthcare Co., Ltd, Kyoto-shi Kyoto 615-0084 (JP); Umeda, Masahiro c/o Omron Healthcare Co., Ltd, Kyoto-shi Kyoto 615-0084 (JP)
(74) Representative: Kilian, Helmut

(56) References cited:
- WO-A-87/06447
- WO-A-02/096287
- WO-A-20/04034902
- US-A- 6 038 469
- US-A1- 2003 013 978
- PATENT ABSTRACTS OF JAPAN vol. 2000, no. 20, 10 July 2001 (2001-07-10) & JP 2001 070267 A (NIPPON KODEN CORP), 21 March 2001 (2001-03-21)

## Description

### BACKGROUND OF THE INVENTION

### Field of the Invention

The present invention relates to a portable electrocardiograph and a processing method, and particularly to improvement in displaying an electrocardiographic waveform representing a measurement result.

### Description of the Background Art

In measuring an electrocardiographic waveform using an electrocardiograph, baseline fluctuation due to a state of a contact between a body and an electrode, body movement of a subject, or the like, is a factor leading to an error in analyzing the electrocardiographic waveform. In particular, in an "event-type electrocardiograph" which a subject anxious about his or her heart carries all the times to detect an electrocardiographic waveform when the subject feels a subjective symptom, it is common to press a measurement electrode detecting an electrical signal against the body surface of the subject to obtain contact therebetween at the time of measuring the electrocardiographic waveform. Accordingly, this technique is more likely to be influenced by the state of the contact between the body and the measurement electrode (a variation in a contact area) or various types of body movements including breathing of the subject, when compared to a technique of attaching or adhering an electrode to the body surface as used in measuring a 12-lead electrocardiogram or a Holter electrocardiogram conducted in a medical institution.

Under such a background, in order to eliminate the influence by the error in analyzing the electrocardiographic waveform due to the baseline fluctuation, numerous technologies have conventionally been proposed, trying to remove the baseline fluctuation itself from the electrocardiographic waveform using an electrical circuit or software processing. Specifically, these technologies try to stabilize the level of a baseline of the electrocardiographic waveform through processing such as filtering a low-frequency component of the electrical signal or adding and averaging a plurality of electrocardiographic waveforms. However, since the electrical signal of the electrocardiographic waveform is very weak, if the processing as described above is performed repeatedly, the electrocardiographic waveform itself might be distorted. Thus, it is more likely that a characteristic waveform showing a condition of a heart will disappear or, on the contrary, an undetected waveform-like characteristic will be erroneously generated, leading to an error in analyzing the electrocardiographic waveform, from a perspective different from the baseline fluctuation. Further, performing such processing requires a larger-sized and more expensive electrocardiograph, which is difficult to be implemented in the event-type electrocardiograph carried by an individual.

Furthermore, analysis of an electrocardiographic waveform is a difficult task even for a highly skilled medical staff member, and numerous technologies for automatically analyzing an electrocardiographic waveform have also been proposed for supporting medical staff. However, in the case of analyzing an electrocardiographic waveform without removing the baseline fluctuation, when a fluctuation large enough to saturate a signal level occurs, the waveform is processed as not normal. Consequently, Japanese Patent Laying-Open No. 06-205752 proposes an electrocardiogram report output device for a Holter electrocardiograph carried by a subject for 24 hours for performing electrocardiography. The electrocardiogram report output device stores electrocardiogram data in which a fluctuation in the signal level is output as an abnormal waveform in real-time analysis, and determines again whether the waveform is normal or abnormal based on the stored electrocardiogram data. Further, Japanese Patent Laying-Open No. 11-206728 proposes a monitor system for a heart. As a technology for advanced analysis and monitoring of myocardial ischemia and infarction, the monitor system has a function of displaying each position of a plurality of electrodes disposed on the body surface of a patient to obtain a plurality of electrical signals and generate ECG (electrocardiogram) data, using a graphic symbol of a portion of the body. The display function of the monitor system has a characteristic of displaying states of the electrical signals from the plurality of electrodes along with the position of each electrode.

However, the technology disclosed in Japanese Patent Laying-Open No. 06-205752 requires the report output device in addition to the Holter electrocardiograph, and without the report output device, it is impossible to determine whether or not an abnormal waveform results from misjudgment due to the baseline fluctuation or the like. This problem has also remained in the "event-type electrocardiograph" which a subject anxious about his or her heart carries all the times to detect an electrocardiographic waveform when the subject feels a subjective symptom. That is, even when an abnormal waveform is detected, such an electrocardiograph does not detect and present whether or not it is due to the baseline fluctuation described above. Consequently, the subject may have needless concern, or a physician may make an analysis error based on the electrocardiographic waveform obtained as a measurement result.

Further, although the states of the electrical signals from the plurality of electrodes can be understood in real time in the technology disclosed in Japanese Patent Laying-Open No. 11-206728, it is impossible for the event-type electrocardiograph, which reads a stored measurement result for analysis, to understand the state of the electrical signal from an electrode at the time of the analysis. Therefore, it is impossible to detect whether or not the waveform indicates abnormality in the electrical signal due to a polarization voltage caused by body movement, which may lead to an analysis error described above.

An electrocardiograph according to the preamble of claim 1, and a processing method formed by an electrocardiograph, according to claim 5 is known from WO-A-02/096287 wherein the processing unit detects a deviation from normal of the electrocardiographic waveform and displays that a deviation from normal has been detected in the electrocardiographic waveform, as the measurement result.

### SUMMARY OF THE INVENTION

The present invention focuses attention on the problems of prior art described above, and an object of the present invention is to prevent an analysis error due to baseline fluctuation in an event-type electrocardiograph carried by a subject at all times.

To achieve the foregoing object, a portable electrocardiograph in accordance with an aspect of the present invention includes an electrode brought into contact with a living body, a processing unit measuring an electrical signal detected by the electrode with being in contact with the living body, as an electrocardiographic waveform, and a display unit displaying a measurement result of the electrocardiographic waveform. The processing unit detects that baseline fluctuation in the electrocardiographic waveform deviates from a predetermined allowable range, and displays on the display unit that the baseline fluctuation deviating from the predetermined allowable range has been detected in the electrocardiographic waveform, as the measurement result.

Preferably, the processing unit distinguishably displays the electrocardiographic waveform for a period during which the baseline fluctuation deviating from the predetermined allowable range has been detected, and also displays the measurement result of the electrocardiographic waveform for all over a measurement period.

Preferably, the processing unit displays the measurement result of the electrocardiographic waveform for a portion of a measurement period, and also distinguishably displays that the electrocardiographic waveform for the portion corresponds to a waveform for a period during which the baseline fluctuation deviating from the predetermined allowable range has been detected.

Preferably, when the processing unit makes a determination that a period during which the baseline fluctuation deviating from the predetermined allowable range has been detected exceeds a predetermined time period, the processing unit displays a result of the determination in addition to the measurement result of the electrocardiographic waveform.

Preferably, the portable electrocardiograph further includes a detachable storage medium, and the processing unit stores in the external storage medium the measurement result of the electrocardiographic waveform together with a detection result that the baseline fluctuation in the electrocardiographic waveform deviates from the allowable range.

According to the present invention, it is detected that baseline fluctuation deviating from a predetermined allowable range, that is, at a level unacceptable for analysis, has occurred during measurement of an electrocardiographic waveform, and such information is displayed for notification. Consequently, by displaying the occurrence of the baseline fluctuation unacceptable for analysis immediately after the measurement, a subject can be urged to make a measurement again. Further, at the time of the analysis based on the measurement result of the electrocardiographic waveform, the electrocardiographic waveform is displayed such that the period during which the baseline fluctuation deviates from the predetermined allowable range can be distinguished, and thus an analysis error on the electrocardiographic waveform during the period can be prevented.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Figs. 1A and 1B are functional block diagrams of a portable electrocardiograph in the present embodiment.
Fig. 2 is a schematic perspective view of the portable electrocardiograph in the present embodiment.
Fig. 3 is a front view of the portable electrocardiograph shown in Fig. 2.
Fig. 4 is a top view of the portable electrocardiograph shown in Fig. 2 when a cover is closed.
Fig. 5 is a bottom view of the portable electrocardiograph shown in Fig. 2 when the cover is closed.
Fig. 6 is a right side view of the portable electrocardiograph shown in Fig. 2.
Fig. 7 is a left side view of the portable electrocardiograph shown in Fig. 2.
Fig. 8 is a perspective view of a measurement posture to be taken by a subject at the time of measuring an electrocardiographic waveform using the portable electrocardiograph in the present embodiment.
Fig. 9 illustrates the measurement posture to be taken by the subject at the time of measuring an electrocardiographic waveform using the portable electrocardiograph in the present embodiment, viewed from the above.
Fig. 10 illustrates an example of a message display screen in accordance with the present embodiment.
Figs. 11A and 11B are processing flow charts in accordance with the present embodiment.
Fig. 12 illustrates an example of a received electrocardiographic waveform in accordance with the present embodiment.
Fig. 13 illustrates a waveform obtained after filtering the electrocardiographic waveform of Fig. 12.
Fig. 14A illustrates an example of a function menu display screen in accordance with the present embodiment.
Fig. 14B illustrates an example of a measurement result list display screen in accordance with the present embodiment.
Figs. 15A and 15B illustrate examples of a measurement result display screen in accordance with the present embodiment.
Fig. 16 illustrates an example of a display mark indicating a period of baseline fluctuation deviating from a predetermined range in accordance with the present embodiment.
Fig. 17 illustrates another example of the display mark indicating the period of the baseline fluctuation deviating from the predetermined range in accordance with the present embodiment.
Figs. 18 to 24 illustrate still other examples of the display mark indicating the period of the baseline fluctuation deviating from the predetermined range in accordance with the present embodiment.

### DESCRIPTION OF THE PREFERRED EMBODIMENTS

In the following, an embodiment of the present invention will be described with reference to the drawings.

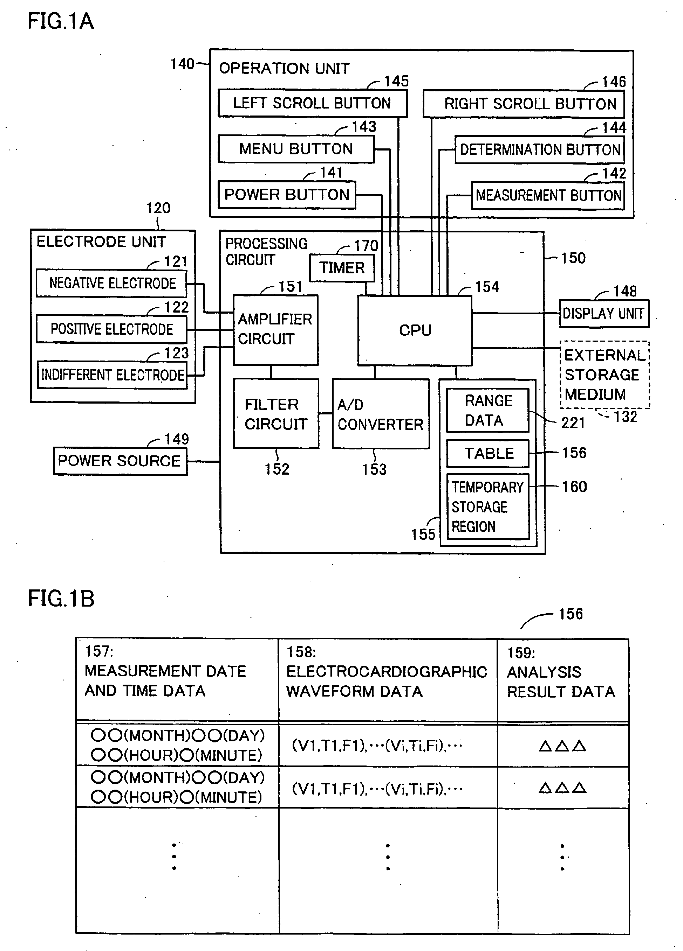

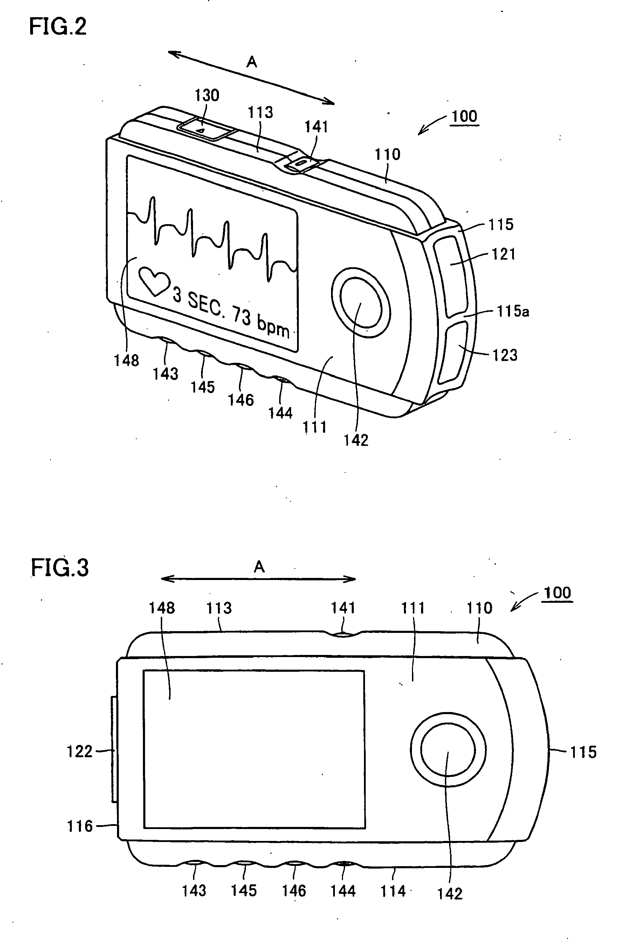

Figs. 1A and 1B are functional block diagrams of a portable electrocardiograph in the present embodiment. Figs. 2 to 7 illustrate an appearance of the portable electrocardiograph in the present embodiment.

### Appearance of the Portable Electrocardiograph

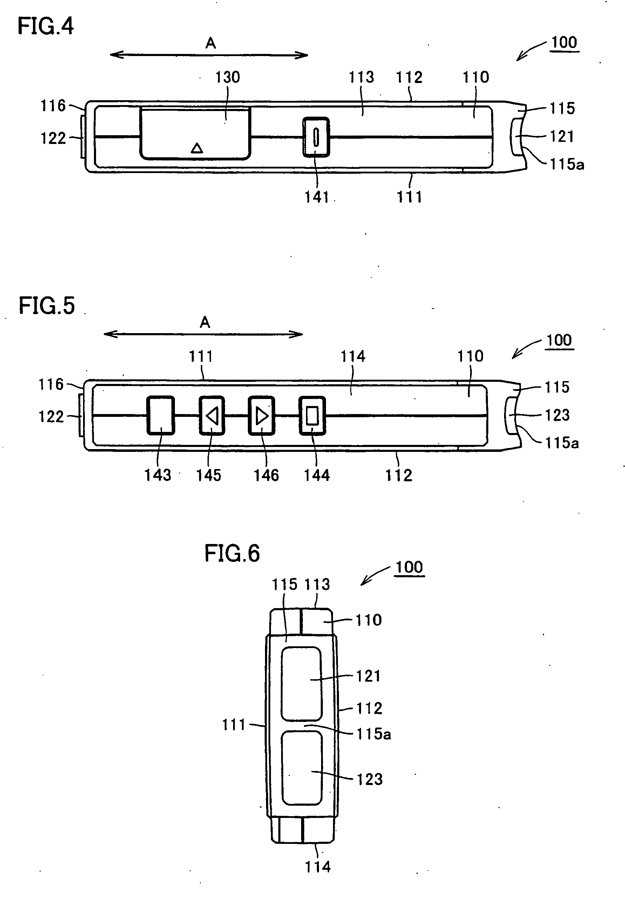

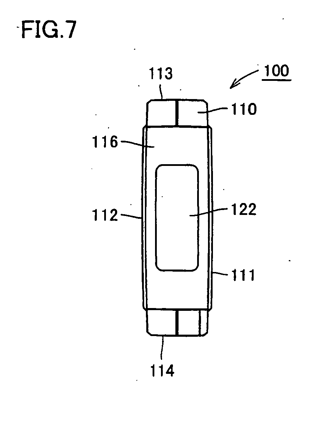

Initially, the appearance of the portable electrocardiograph in the present embodiment will be described. Fig. 2 is a schematic perspective view of the portable electrocardiograph in the present embodiment of the invention, and Fig. 3 is a front view of the portable electrocardiograph shown in Fig. 2. Figs. 4 and 5 are a top view and a bottom view, respectively, of the portable electrocardiograph shown in Fig. 2 when a cover is closed. Figs. 6 and 7 are a right side view and a left side view, respectively, of the portable electrocardiograph shown in Fig. 2.

As shown in Figs. 2 to 7, in order to realize excellent usability, a portable electrocardiograph 100 in the present embodiment has such a light weight and a small size that it can be held with one hand. Portable electrocardiograph 100 includes a device main body 110 having a flat and elongated, substantially rectangular parallelepiped outer shape. On its outer surfaces (a front face 111, a rear face 112, a top face 113, a bottom face 114, a right side face 115, and a left side face 116), a display unit, an operation unit, a measurement electrode, and the like are disposed.

As shown in Figs. 2 and 3, a measurement button 142 serving as an operation button for starting measurement is provided in a portion close to one end in the longitudinal direction (the direction shown with an arrow A in the drawing) of front face 111 of device main body 110. In a portion close to the other end of front face 111 of device main body 110, a display unit 148 is provided. Display unit 148 is implemented, for example, by a liquid crystal display and serves to display a result of measurement or the like. The measurement result is displayed, for example, as an electrocardiographic waveform or numerical data as shown in Fig. 2.

As shown in Figs. 2 and 4, a power button 141 is disposed in a predetermined position on top surface 113 of device main body 110. Power button 141 serves as an operation button for turning ON/OFF portable electrocardiograph 100. A cover 130 is provided in a predetermined position on top face 113 of device main body 110. Cover 130 is provided in order to cover a slot for an external storage medium (not shown) when it is closed, and cover 130 is attached to device main body 110 such that it is freely opened and closed.

As shown in Figs. 2 and 5, various operation buttons are disposed in predetermined positions on bottom face 114 of device main body 110. In shown portable electrocardiograph 100, a menu button 143, a determination button 144, a left scroll button 145, and a right scroll button 146 are disposed. Menu button 143 is used for displaying various menus of portable electrocardiograph 100, while determination button 144 is used for performing a menu or each operation. Left scroll button 145 and right scroll button 146 are used for scrolled display of a graph showing a measurement result or guide information displayed on display unit 148.

As shown in Figs. 2 and 6, on right side face 115 located at one end in the longitudinal direction of device main body 110, a negative electrode 121 representing one electrode out of a pair of measurement electrodes, as well as an indifferent electrode 123 for deriving a potential serving as a reference in potential variation in the body are disposed. Right side face 115 has a smoothly curved shape, such that a forefinger of the right hand of the subject is fitted thereto when the subject takes a measurement posture which will be described later. In addition, a concave portion 115a extending in an up-down direction is formed in right side face 115. Concave portion 115a is in a shape to receive the forefinger of the right hand of the subject.

Negative electrode 121 and indifferent electrode 123 described above are formed with a conductive member, and disposed in concave portion 115a provided in right side face 115 such that their surfaces are exposed on the outer surface of device main body 110. Negative electrode 121 is located closer to top face 113 on right side face 115, while indifferent electrode 123 is located closer to bottom face 114 on right side face 115. On left side face 116 located at the other end in the longitudinal direction of device main body 110, a positive electrode 122 representing the other electrode out of the pair of measurement electrodes is disposed.

### Posture for Measurement

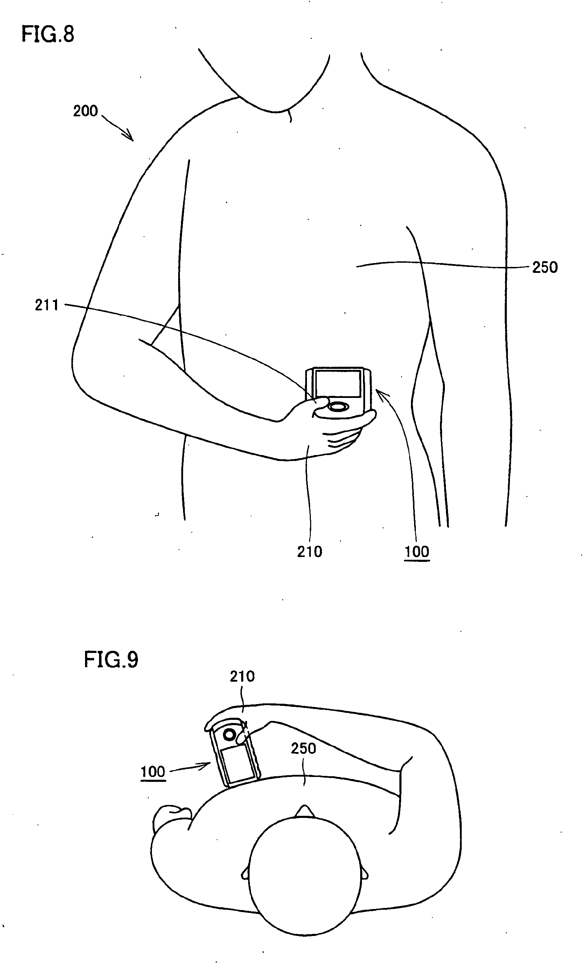

Referring to Figs. 8 and 9, a posture in measuring an electrocardiographic waveform using portable electrocardiograph 100 will be described. A subject 200 holds a portion closer to one end of device main body 110 of portable electrocardiograph 100 with his/her right hand 210, and brings positive electrode 122 provided on left side face 116 located at the other end of device main body 110 into direct contact with the skin on a fifth intercostal anterior axillary line located in a lower left portion of a chest 250. Then, the subject presses measurement button 142 provided on front face 111 of device main body 110 with his/her thumb 211 of right hand 210, and maintains this state for several tens of seconds until measurement of the electrocardiographic waveform is completed.

### Processing Circuit of the Portable Electrocardiograph

As shown in Fig. 1A, device main body 110 contains a processing circuit 150 for processing a bioelectric signal detected by the measurement electrode to be measured as an electrocardiographic waveform. Processing circuit 150 includes, for example, an amplifier circuit 151 amplifying the bioelectric signal detected by the measurement electrode, a filter circuit 152 removing a noise component from the bioelectric signal detected by the measurement electrode, an A/D (analog/digital) converter 153 converting an analog signal to a digital signal, a CPU (central processing unit) 154 performing various computations, a memory 155 having a ROM (Read Only Memory) or a RAM (Random Access Memory) storing electrocardiographic information, and a timer 170 performing timing operation and outputting timed time data to CPU 154. Amplifier circuit 151 performs differential amplification for voltage signals (bioelectric signals) output from negative electrode 121 and positive electrode 122 based on a voltage signal output from indifferent electrode 123, for output. Filter circuit 152 is for example a band pass filter having a pass band ranging from 0.5 Hz to 35 Hz.

To processing circuit 150 are connected an electrode unit 120 having negative electrode 121, positive electrode 122, and indifferent electrode 123 described above; an operation unit 140 having power button 141, measurement button 142, menu button 143, determination button 144, left scroll button 145, and right scroll button 146; a display unit 148; and a power source 149. In addition, an external storage medium 132 inserted into the slot for the external storage medium (not shown) is also connected to processing circuit 150.

### Example of Contents Stored in Memory

Memory 155 stores a table 156 shown in Fig. 1B for storing electrocardiographic information as a plurality of measurement results. Table 156 stores electrocardiographic waveform data 158 representing an electrocardiographic waveform, analysis result data 159 representing an analysis result of the waveform, and measurement date and time data 157 representing a measurement date and time (a date and time when a measurement is started or completed), which are related to each other. The contents of table 156 are displayed on display unit 148, and also transferred to external storage medium 132 to be stored therein. Further, memory 155 stores a temporary storage region 160 and range data 221 representing a predetermined range for determining a level of a baseline.

Electrocardiographic waveform data 158 in table 156 includes a plurality of sets (Vi, Ti, Fi) (where i=1, 2, 3, ...) obtained through periodical measurements, in which Vi is amplitude (voltage) level data of an electrocardiographic waveform, Ti is measurement time data, and Fi is a noise flag.

### Measurement Operation of the Processing Circuit

In processing circuit 150 of portable electrocardiograph 100, when the subject presses measurement button 142 in the state shown in Fig. 8 or Fig. 9, the pressing causes an instruction to start measuring an electrocardiographic waveform to be provided to CPU 154. Then, CPU 154 shifts to a state of starting processing such as making an analysis on a signal received from A/D converter 153.

An electrical signal detected by electrode unit 120 in the state shown in Fig. 8 or Fig. 9 is supplied to amplifier circuit 151. Amplifier circuit 151 receives and amplifies the supplied electrical signal, and supplies the amplified electrical signal to filter circuit 152. Filter circuit 152 receives the supplied electrical signal, removes a noise component from the signal to be acceptable for processing in a circuit in a later stage, and supplies the signal to A/D converter 153. A/D converter 153 receives the supplied analog electrical signal (bioelectric signal) and converts the received signal to a digital signal for output to CPU 154.

CPU 154 receives the digital signal supplied from A/D converter 153, and displays on display unit 148 a waveform based on the electrocardiographic waveform data obtained from real-time analysis. In addition, CPU 154 temporarily stores the waveform in temporary storage region 160 in a RAM within memory 155. The analysis by CPU 154 refers to processing for detecting the presence or absence of a characteristic with respect to a shape representing such as arrhythmia or myocardial ischemia, a characteristic with respect to a period representing such as bradycardia or tachycardia, or a waveform which cannot be analyzed due to noise, baseline fluctuation, or the like, based on the electrocardiographic waveform in the form of the digital signal, and analyzing a detected result. The result of the analysis corresponds to analysis result data 159. The analysis procedure follows a known procedure.

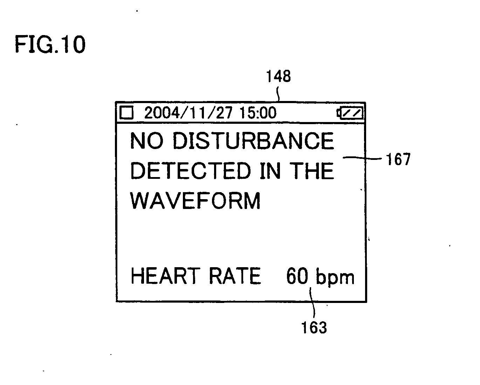

When the measurement of the electrocardiographic waveform is completed, CPU 154 displays on display unit 148 a message asking the subject whether or not to store the electrocardiographic waveform temporarily stored in the RAM into table 156. If the subject reads the message and performs an operation via operation unit 140 to provide an instruction to store the waveform, the instruction designated by the operation is provided to CPU 154. In response to receiving the instruction to store the waveform, CPU 154 stores current date and time data, and the electrocardiographic waveform data and the analysis result temporarily stored in temporary storage region 160, into table 156 of memory 155, with relating them each other as measurement date and time data 157, electrocardiographic waveform data 158, and analysis result data 159. Then, CPU 154 edits the analysis result of the electrocardiographic waveform into a message and displays the message on display unit 148 as shown in Fig. 10. In Fig. 10, data 163 on a heart rate per unit time is displayed along with a message 167. The heart rate can be obtained by a known procedure, based on the electrocardiographic waveform.

On the other hand, when the instruction for storage is not provided, CPU 154 discards the data on the electrocardiographic waveform and the analysis result temporarily stored in temporary storage region 160.

### Detection and Processing of Noise and Baseline Fluctuation

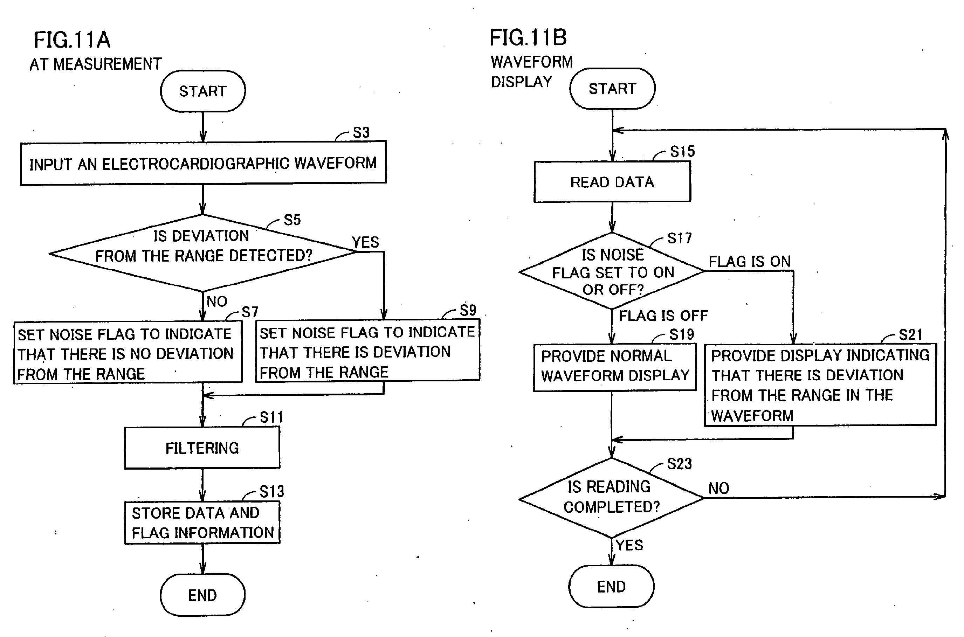

While various analyses are performed as described above in the present embodiment, here it is assumed for the sake of clarity that the presence or absence of a waveform which cannot be analyzed due to noise or baseline fluctuation is detected and the waveform, if present, is processed. A procedure for the detection and the processing will be described with reference to Fig. 11A. The flow chart in Fig. 11A has been stored in memory 155 as a program, and CPU 154 reads the program from memory 155 for execution.

Firstly, when CPU 154 receives a digital signal representing an electrocardiographic waveform from A/D converter 153 (in Step S (hereinafter will simply be abbreviated to S) 3), as for an electrocardiographic waveform signal (electrical signal) received in chronological order in accordance with time Ti output from timer 170, CPU 154 detects whether or not a voltage level recognized as a baseline by a known predetermined procedure deviates from a predetermined range in which analysis of the waveform is allowable (S5). Specifically, CPU 154 compares the voltage level with the predetermined range designated by range data 221 to determine whether or not the voltage level deviates from the predetermined range. If CPU 154 does not detect deviation from the range (NO in S5), noise flag Fi representing the deviation from the range is set to OFF (S7), and if CPU 154 detects the deviation from the range (YES in S5), noise flag Fi is set to ON (S9). Thereafter, filtering is performed on the electrocardiographic waveform signal (S11). Then, CPU 154 stores in table 156 the plurality of sets (Vi, Ti, Fi) each having noise flag Fi, data Vi of the electrocardiographic waveform after filtering, and measurement time data Ti received from timer 170, as electrocardiographic waveform data 158, with relating it to measurement date and time data 157 representing when the measurement is started or completed (S 13). Specifically, when CPU 154 asks the subject and receives the instruction for storage, CPU 154 stores electrocardiographic waveform data 158 in table 156, but otherwise it discards the data. Electrocardiographic waveform data 158 in table 156 includes data Vi of an amplitude level in chronological order of the electrical signal obtained in a measurement period of a predetermined length, and noise flag Fi for amplitude level data Vi, which are related to each other. When noise flag Fi is ON, it indicates that the corresponding amplitude level data Vi deviates from the range designated by range data 221, and when noise flag Fi is OFF, it indicates that amplitude level data Vi does not deviate from that range.

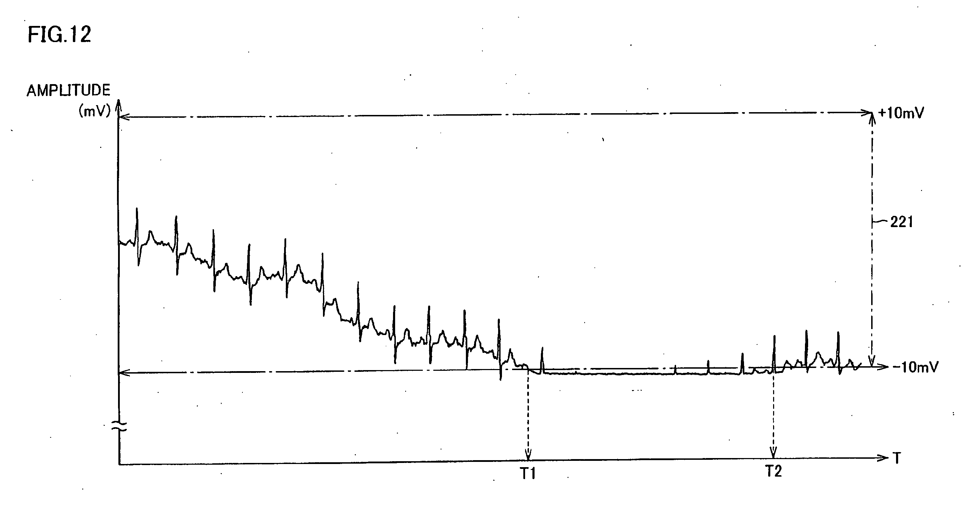

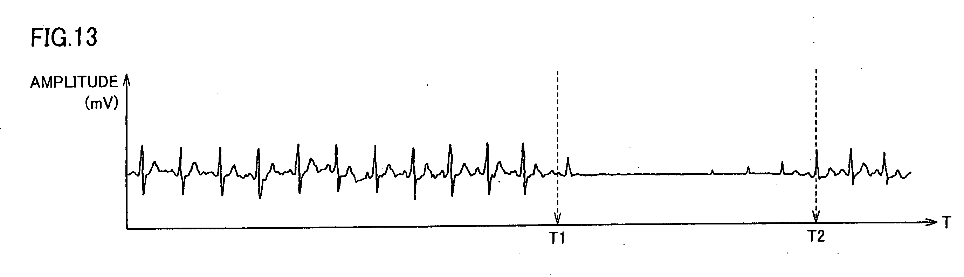

Referring to Figs. 12 and 13, the detection of the deviation from the range in S5 and the filtering will be described. Each of Figs. 12 and 13 plots an electrocardiographic waveform, in which the vertical axis represents an amplitude (mV) of the electrocardiographic waveform and the horizontal axis represents elapsed time T for measurement. Fig. 12 illustrates the electrocardiographic waveform supplied from A/D converter 153 to CPU 154, and Fig. 13 illustrates the electrocardiographic waveform obtained after filtering the electrocardiographic waveform of Fig. 12. Fig. 12 shows an example where baseline fluctuation at a level unacceptable for analysis occurs in the electrocardiographic waveform. This example represents the case where the level of the baseline falls below a lower limit of the predetermined range designated by range data 221 due to unstable contact between positive electrode 122 and chest 250 of subject 200 during the measurement of the electrocardiographic waveform.

While noise flag Fi is set to 'ON' or 'OFF' indicating whether or not the amplitude level of the electrocardiographic waveform deviates from the predetermined range in the foregoing, it is assumed that, in the description of Figs. 12 and 13, noise flag Fi is set to '00' when the level does not deviate from the predetermined range, '01' when the level exceeds an upper limit of the predetermined range, and '11' when the level falls below a lower limit of the predetermined range.

Provided that the electrocardiographic waveform of Fig. 12 is supplied for the processing in S5, CPU 154 determines whether or not amplitude level Vi of the electrocardiographic waveform supplied per time Ti over the course of time T falls within the predetermined range designated by range data 221 (for example, from -10 mV to +10 mV). In this case, CPU 154 detects that amplitude level Vi falls below the lower limit of the predetermined range in a period from time T1 to time T2 (YES in S5), and thus, for the electrocardiographic waveform, CPU 154 sets '11' as a value for noise flag Fi to indicate that amplitude level Vi falls below the lower limit of the predetermined range in the period from time T1 to time T2 (S9). Thereafter, in the filtering in S 11, CPU 154 removes a noise component overlapping the electrical signal of the electrocardiographic waveform. In the removal of the noise component, predetermined levels of a high-frequency component and a low-frequency component in the electrical signal of the electrocardiographic waveform are removed, and during a period in which the level of the baseline deviates from the predetermined range designated by range data 221, a component in the electrical signal of the electrocardiographic waveform deviating from the predetermined range is removed. In the latter removal, the level of the electrical signal of the electrocardiographic waveform during a period in which the level exceeds an upper limit level of the predetermined range is replaced by the upper limit level, and the level of the signal during a period in which the level falls below a lower limit level of the predetermined range is replaced by the lower limit level. As a result, the waveform of Fig. 12 becomes the one as shown in Fig. 13.

It is to be noted that, when CPU 154 determines that the period from time T1 to time T2 in which the electrocardiographic waveform cannot be analyzed due to the deviation from the range exceeds a predetermined period (for example, half the total measurement time of a predetermined length), CPU 154 determines that the period in which analysis cannot be performed due to baseline fluctuation exceeds the predetermined period, and displays on display unit 148 a message urging the subject to calm down to suppress body movement and make a measurement again. Thereby, CPU 154 can urge the subject to take a measurement posture not to cause baseline fluctuation. Consequently, the electrocardiographic waveform when an event occurs can be measured again as a waveform capable of being analyzed more reliably.

### Reading and Displaying of Measurement Result

Portable electrocardiograph 100 has a function of reading the measurement and analysis result of the electrocardiographic waveform stored in table 156 and displaying the result on display unit 148. Here, assume that table 156 stores a plurality of electrocardiographic waveform data 158 in advance.

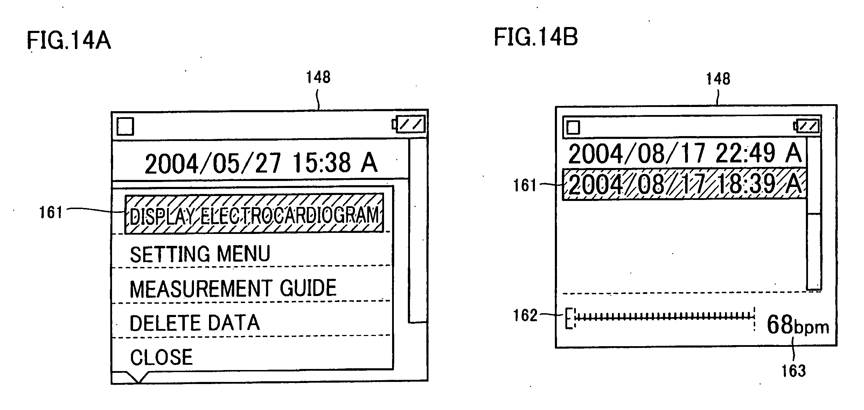

If the subject presses menu button 143 when portable electrocardiograph 100 is powered ON, CPU 154 displays on display unit 148 a function menu screen shown in Fig. 14A. A cursor 161 highlighted on the screen moves in conjunction with the operation of left scroll button 145 or right scroll button 146. The subject operates left scroll button 145 or right scroll button 146 while checking the screen, moves cursor 161 to a position indicating "Display Electrocardiogram", which is a desired item in the function menu, and then presses determination button 144.

When CPU 154 detects that determination button 144 has been pressed with "Display Electrocardiogram" indicated, CPU 154 reads measurement date and time data 157 from table 156 in memory 155, and displays the data as a list on display unit 148. Fig 14B illustrates an example of the display screen on this occasion.

When the subject operates left scroll button 145 or right scroll button 146 while checking the screen of Fig. 14B, cursor 161 moves in conjunction with the operation, indicating measurement date and time data 157 in the list on the screen in turn. Every time each measurement date and time data 157 is indicated by cursor 161, CPU 154 . reads from table 156 electrocardiographic waveform data 158 corresponding to the indicated measurement date and time data 157, and displays an entire waveform as a reduced waveform 162 on a lower portion of the screen, based on the read electrocardiographic waveform data 158. Further, data 163 representing the heart rate obtained based on electrocardiographic waveform data 158 is also displayed.

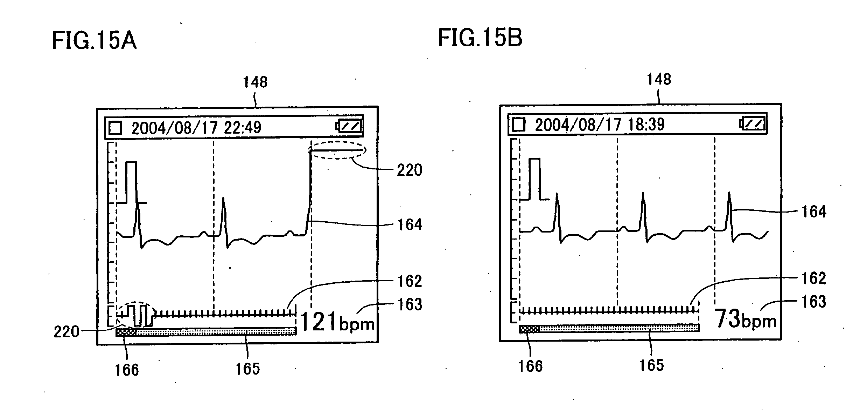

Since the subject can specify a desired electrocardiographic waveform by checking reduced waveform 162 displayed during scrolling, the subject presses determination button 144 when the desired reduced waveform 162 is displayed. Upon detecting the pressing of determination button 144, CPU 154 reads from table 156 electrocardiographic waveform data 158 corresponding to measurement date and time data 157 indicated by cursor 161 at that time, and displays on display unit 148 information based on the read electrocardiographic waveform data 158. Figs. 15A and 15B illustrate examples of the display screen on this occasion.

On each screen of Figs. 15A and 15B, at the start of the display, reduced waveform 162 representing the entire waveform over the total measurement period is displayed on the lower portion of the screen, and an enlarged waveform 164 representing the waveform for two minutes after the start of the measurement is displayed on its upper portion. Further, a scale bar 165 showing the length of the measurement period is displayed immediately below reduced waveform 162. On scale bar 165 is displayed a pointer 166 for indicating to which portion of the waveform in the total measurement period enlarged waveform 164 corresponds. The portion of the waveform in the total measurement period to be shown as enlarged waveform 164 can be changed in units of two seconds. Specifically, it can be changed in units of two seconds by operating right scroll button 146 and left scroll button 145 to move pointer 166 on scale bar 165.

When the subject presses determination button 144 after checking the electrocardiographic waveform on the screens of Figs. 15A and 15B, CPU 154 reads from table 156 analysis result data 159 corresponding to the displayed electrocardiographic waveform data 158, edits a message based on the read analysis result data 159, and displays the edited message on display unit 148 for example as shown in Fig. 10. When the subject presses determination button 144 again in this state, CPU 154 detects that determination button 144 has been pressed and moves the display screen from the one in Fig. 10 back to the one in Fig. 14B.

### Procedure for Displaying Measured Electrocardiographic Waveform

Although the read and displayed electrocardiographic waveform in the display example of Fig. 15B does not include baseline fluctuation at a level unacceptable for analysis, the displayed electrocardiographic waveform in the display example of Fig. 15A includes baseline fluctuation at a level unacceptable for analysis. In such a case, during the period in which analysis cannot be performed due to the baseline fluctuation, mark 220 is displayed in reduced waveform 162 and enlarged waveform 164 in place of the electrocardiographic waveform. Mark 220 indicates the period in which analysis cannot be performed due to the baseline fluctuation. A procedure for displaying thereof will be described following the flow chart shown in Fig. 11B. The flow chart of Fig. 11B has been stored in memory 155 as a program, and CPU 154 reads the program from memory 155 for execution. Assume that noise flag Fi is set to 'ON' or 'OFF' in accordance with the procedure described above.

Firstly, at the time of displaying an electrocardiographic waveform selected in the list of Fig. 14B, CPU 154 reads the selected electrocardiographic waveform data 158 from table 156 (S15). CPU 154 then detects to which of 'ON' and 'OFF' noise flag Fi is set for the read electrocardiographic waveform data 158 (S17). When CPU 154 detects that no noise flag Fi is set to 'ON' for electrocardiographic waveform data 158 (flag is 'OFF' in S17), CPU 154 provides normal waveform display based on the read electrocardiographic waveform data 158. Specifically, two-second enlarged waveform 164 (the waveform during the period indicated by pointer 166) and reduced waveform 162 are displayed without mark 220 as shown in Fig. 15B.

On the contrary, when CPU 154 detects noise flag Fi which is set to 'ON' for the read electrocardiographic waveform data 158 (flag is 'ON' in S17), CPU 154 displays reduced waveform 162 with mark 220 as well as enlarged waveform 164 as shown in Fig. 15A, based on the read electrocardiographic waveform data 158 (S21). Enlarged waveform 164 displayed on this occasion is a waveform in a two-second period indicated by pointer 166. If the data for the two-second period in electrocardiographic waveform data 158 has a partial data in which noise flag Fi is set to 'ON', mark 220 is displayed in place of the waveform corresponding to the partial data in enlarged waveform 164.

When pointer 166 on the screen is moved by operation after the displaying step in S 19 or S21 to indicate to display another two-second enlarged waveform 164, CPU 154 does not determine that the reading of electrocardiographic waveform data 158 is to be completed (the displaying is to be completed) (NO in S23), and the control passes back to S 15 in which CPU 154 reads electrocardiographic waveform data 15 8 from table 156 and performs the later processing steps from S 17 to S23 as described above. On the other hand, if the subject operates operation unit 140 to input a predetermined instruction to complete the displaying, CPU 154 determines that the reading of electrocardiographic waveform data 158 is to be completed (the displaying is to be completed) (YES in S23), and completes the processing.

By providing the displaying and the processing in this manner, a characteristic which will be described below can be obtained. Specifically, it is assumed that, due to a considerable drop in the level of the baseline during the period from time T 1 to time T2 as shown in Fig. 12, the level deviates from the predetermined range designated by range data 221, and a saturated waveform is measured during the period as shown in Fig. 13 and stored in table 156. In this case, if the electrocardiographic waveform data is read from table 156 and displayed as it is, the waveform which should be displayed is not displayed for several heart rates during the period from time T1 to time T2. In this manner, if the displayed electrocardiographic waveform is analyzed with no information indicating the occurrence of baseline fluctuation presented, this may lead to a misdiagnosis that the subject had bradycardia or atrial fibrillation during that period. In the present embodiment, however, the electrocardiographic waveform during that period is displayed with mark 220 indicating the occurrence of the baseline fluctuation deviating from the predetermined range, and thus misdiagnosis as described above can be avoided.

### Display Examples of the Mark

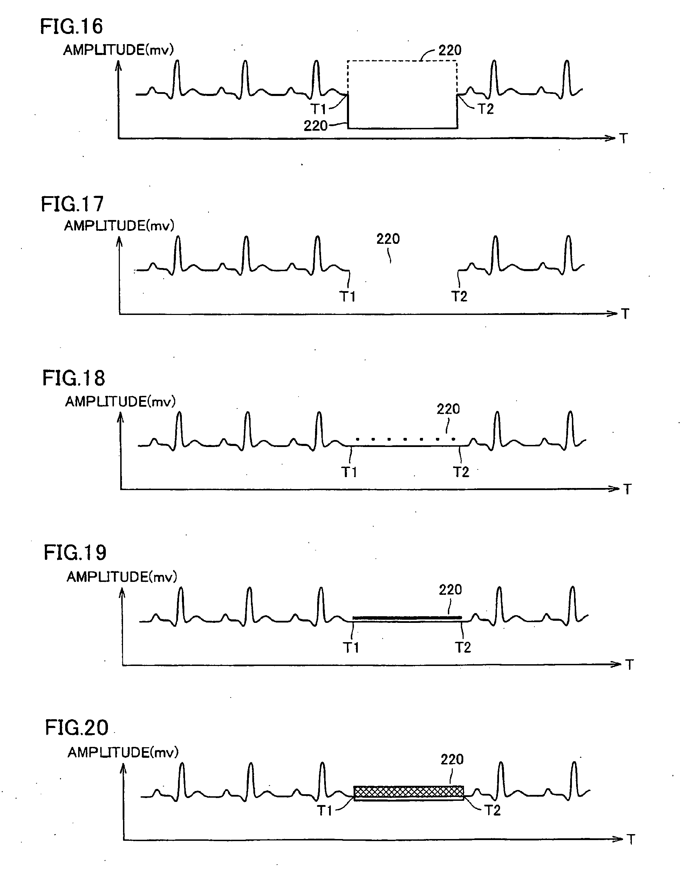

Figs. 16 to 24 illustrate various examples of mark 220 indicating the period of the baseline fluctuation from time T1 to time T2 described above. In these drawings, the electrocardiographic waveforms are illustrated schematically.

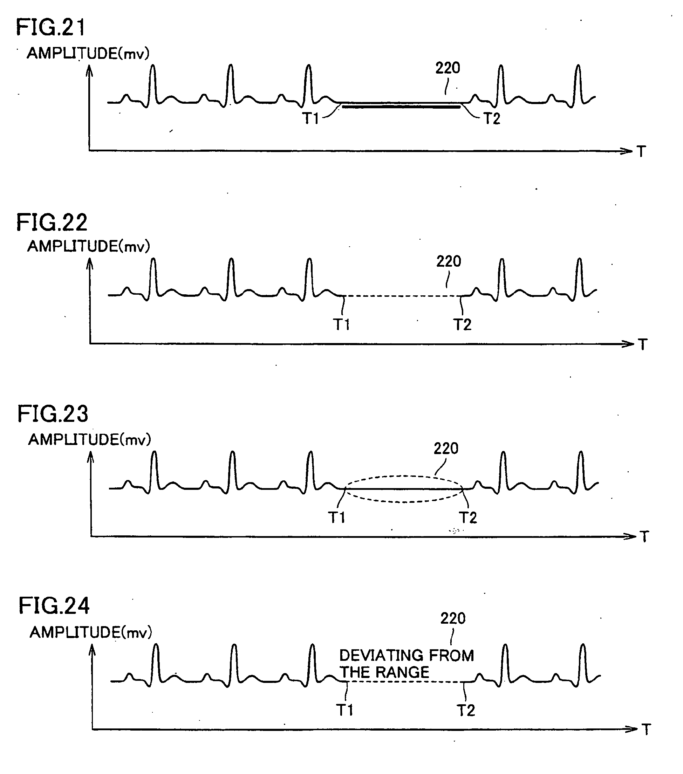

As one display example of mark 220, firstly it may be a rectangular wave as shown in Fig. 16. In Fig. 16, if the level falls below the lower limit of the range, mark 220 is a rectangular wave falling downward, and if the level exceeds the upper limit of the range, mark 220 is a rectangular wave rising upward. Further, mark 220 for the period may be represented as a space, as shown in Fig. 17. Mark 220 may also be displayed as a straight line drawn over the period with dots added above the straight line as shown in Fig. 18, or the dots may be replaced by a thick solid line as shown in Fig. 19. Fig. 21 illustrates a case where the thick solid line drawn above the straight line in Fig. 19 is drawn below the straight line instead to display mark 220.

Further, mark 220 may be displayed with hatching overlying the straight line drawn over the period as shown in Fig. 20. Mark 220 may also be displayed with the straight line drawn over the period surrounded by a dashed line as shown in Fig. 23. Instead of drawing the foregoing solid straight lines over the period, a dashed straight line may be drawn as shown in Figs. 22 and 24. Furthermore, as shown in Fig. 24, a message indicating the deviation from the range is added to the line drawn over the period to specifically indicate that the level of the baseline deviates from the range during the period.

### Data Transfer

Portable electrocardiograph 100 also has a function of transferring the measurement and analysis result of the electrocardiographic waveform to external storage medium 132 to store the result therein. Thereby, by removing from portable electrocardiograph 100 external storage medium 132 storing the data measured and analyzed in portable electrocardiograph 100 and setting the medium to an external computer terminal, the computer terminal can read the measured and analyzed data from the set external storage medium 132, and process the data as in the present embodiment for display. Off course, also in this case, misdiagnosis due to baseline fluctuation can be avoided even when the measurement and analysis result of the electrocardiographic waveform is used in a device other than portable electrocardiograph 100 such as an external computer terminal, by storing the measurement and analysis result of the electrocardiographic waveform in external storage medium 132 together with information such as whether or not baseline fluctuation at a level unacceptable for analysis is included, and if any, during which period it is included.

## Claims

1. A portable electrocardiograph (100), comprising:
an electrode (120) brought into contact with a living body (250),
a processing unit (150) measuring an electrical signal detected by said electrode (120) being in contact with said living body (250), as an electrocardiographic waveform, and
a display unit (148) displaying a measurement result of the electrocardiographic waveform wherein
said processing unit (150) detects that baseline fluctuation in the electrocardiographic waveform deviates from a predetermined allowable range (221) (S5), and displays on said display unit (148) that the baseline fluctuation deviating from said predetermined allowable range (221) has been detected in the electrocardiographic waveform, as said measurement result, **characterized in that**
said processing unit (150) distinguishably displays the electrocardiographic waveform for a period during which the baseline fluctuation deviating from said predetermined allowable range (221) has been detected, and also displays the measurement result of the electrocardiographic waveform for all over a measurement period.

2. The portable electrocardiograph (100) according to claim 1, wherein
said processing unit (150) displays the measurement result of the electrocardiographic waveform for a portion of a measurement period, and also distinguishably displays that the electrocardiographic waveform for the portion corresponds to a waveform for a period during which the baseline fluctuation deviating from said predetermined allowable range (221) has been detected.

3. The portable electrocardiograph (100) according to claim 1, wherein
when said processing unit (150) makes a determination that a period during which the baseline fluctuation deviating from said predetermined allowable range (221) has been detected exceeds a predetermined time period, said processing unit (150) displays a result of the determination in addition to the measurement result of the electrocardiographic waveform.

4. The portable electrocardiograph (100) according to claim 1, further comprising a detachable storage medium (132), wherein
said processing unit (150) stores in said external storage medium (132) the measurement result of the electrocardiographic waveform together with a detection result that the baseline fluctuation in the electrocardiographic waveform deviates from the allowable range (221).

5. A processing method performed by a portable electrocardiograph having an electrode (120) brought into contact with a living body (250) and a display unit (148) displaying a measurement result of an electrocardiographic waveform, comprising a processing step of measuring an electrical signal detected by said electrode (120) being in contact with said living body (250) as an electrocardiographic waveform,
detecting that baseline fluctuation in the electrocardiographic waveform deviates from a predetermined allowable range (221) (S5),
displaying on said display unit (148) that the baseline fluctuation deviating from said predetermined allowable range (221) has been detected in the electrocardiographic waveform, as said measurement result **characterized in that** said processing step includes the step of distinguishably
displaying the electrocardiographic waveform for a period during which the baseline fluctuation deviating from said predetermined allowable range (221) has been detected, and also displaying the measurement result of the electrocardiographic waveform for all over a measurement period.

## Patentansprüche

1. Tragbarer Elektrocardiograph (100), welcher aufweist:
eine mit einem Lebewesen (250) in Berührung gebrachte Elektrode (120),
eine Verarbeitungseinheit (150), welche ein elektrisches Signal, das mit der mit dem Lebewesen (250) in Berührung stehenden Elektrode (120) festgestellt wird, als elektrocardiographischen Schwingungsverlauf misst, und
eine Anzeigeeinheit (148), welche ein Messergebnis für den elektrocardiographischen Schwingungsverlauf anzeigt, wobei
die Verarbeitungseinheit (150) feststellt, dass eine Grundlinienschwankung des elektrocardiograpriischen Schwingungsverlaufs von einem bestimmten zulässigen Bereich (221) abweicht (S5) und auf der Anzeigeeinheit (148) als das Messergebnis anzeigt, dass die von dem bestimmten zulässigen Bereich (221) abweichende grundlegende Schwankung in dem elektrocardiographischen Schwingungsverlauf festgestellt worden ist, **dadurch gekennzeichnet, dass**
die Verarbeitungseinheit (150) unterscheidbar den elektrocardiographischen Schwingungsverlauf für eine Zeitdauer anzeigt, während welcher die von dem bestimmten zulässigen Bereich (221) abweichende Grundlinienschwankung festgestellt worden ist, und auch das Messergebnis für den elektrocardiographischen Schwingungsverlauf für eine gesamte Messzeitdauer anzeigt.

2. Tragbarer Elektrocardiograph (100) nach Anspruch 1, wobei
die Verarbeitungseinheit (150) das Messergebnis für den elektrocardiographischen Schwingungsverlauf für einen Abschnitt einer Messzeitdauer anzeigt und auch unterscheidbar anzeigt, dass der elektrocardiographische Schwingungsverlauf für den Abschnitt einem Schwingungsverlauf für eine Zeitdauer entspricht, während welcher die von dem bestimmten zulässigen Bereich (221) abweichende Grundlinienschwankung festgestellt worden ist.

3. Tragbarer Elektrocardiograph (100) nach Anspruch 1, wobei,
wenn die Verarbeitungseinheit (150) eine Feststellung trifft, dass eine Zeitdauer, während welcher die von dem bestimmten zulässigen Bereich (221) abweichende Grundlinienschwankung festgestellt worden ist, eine bestimmte Zeitdauer überschreitet, die Verarbeitungseinheit (150) ein Ergebnis der Feststellung zusätzlich zum Messergebnis für den elektrocardiographischen Schwingungsverlauf anzeigt.

4. Tragbarer Elektrocardiograph (100) nach Anspruch 1, welcher ferner ein abnehmbares Speichermedium (132) aufweist, wobei
die Verarbeitungseinheit (150) in dem externen Speichermedium (132) das Messergebnis für den elektrocardiographischen Schwingungsverlauf zusammen mit einem Feststellungsergebnis, das die Grundlinienschwankung des elektrocardiographischen Schwingungsverlaufs von dem zulässigen Bereich (221) abweicht, speichert.

5. Verarbeitungsverfahren, welches von einem tragbaren Elektrocardiographen mit einer Elektrode (120), die mit einem Lebewesen (250) in Berührung gebracht ist, und mit einer Anzeigeeinheit (148), die ein Messergebnis eines elektrocardiographischen Schwingungsverlaufs anzeigt, durchgeführt wird, wobei das Verfahren eine Verarbeitungsschritt der Messung eines mit der Elektrode (120), welche mit dem Lebewesen (250) in Berührung gebracht ist, festgestellten elektrischen Signals als elektrocardiographischen Schwinungsverlauf,
des Feststellens, dass eine Grundlinienschwankung des elektrocardiographischen Schwingungsverlaufs von einem bestimmten zulässigen Bereich (221) abweicht (S5),
des Anzeigens auf der Anzeigeeinheit (148), dass die von dem bestimmten zulässigen Bereich (221) abweichende Grundlinienschwankung in dem elektrocardiographischen Schwingungsverlauf festgestellt worden ist, als Messergebnis aufweist, **dadurch gekennzeichnet, dass** der Verarbeitungsschritt den Schritt des unterscheidbaren Anzeigens des elektrocardiographischen Schwingungsverlaufs für eine Zeitdauer, während der die von dem bestimmten zulässigen Bereich (221) abweichende Grundlinienschwankung festgestellt worden ist, und auch des Anzeigens des Messergebnisses des elektrocardiographischen Schwingungsverlaufs für eine gesamte Messzeitdauer enthält.

## Revendications

1. Électrocardiographe portable (100), comprenant :
■ une électrode (120) mise en contact avec un corps vivant (250),
■ une unité de traitement (150) mesurant un signal électrique détecté par ladite électrode (120) en contact avec ledit corps vivant (250), sous la forme d'une forme d'onde électrocardiographique, et
■ une unité d'affichage (148) affichant un résultat de mesure de la forme d'onde électrocardiographique, dans lequel
■ ladite unité de traitement (150) détecte qu'une fluctuation de base dans la forme d'onde électrocardiographique dévie de la plage autorisée prédéterminée (221) (S5), et
affiche sur ladite unité d'affichage (148) que la fluctuation de base déviant de ladite plage autorisée prédéterminée (221) a été détectée dans la forme d'onde électrocardiographique, sous la forme dudit résultat de mesure, **caractérisé en ce que**
■ ladite unité de traitement (150) affiche de façon distinguable la forme d'onde électrocardiographique pendant une période durant laquelle la fluctuation de base déviant de ladite plage autorisée prédéterminée (221) a été détectée, et affiche également le résultat de mesure de la forme électrocardiographique sur l'ensemble d'une période de mesure.

2. Électrocardiographe portable (100) selon la revendication 1, dans lequel ladite unité de traitement (150) affiche le résultat de mesure de la forme d'onde électrocardiographique sur une partie de la période de mesure, et affiche également de façon distinguable que la forme d'onde électrocardiographique sur cette partie correspond à la forme d'onde pendant une période durant laquelle la fluctuation de base déviant de ladite plage autorisée prédéterminée (221) a été détectée.

3. Électrocardiographe portable (100) selon la revendication 1, dans lequel lorsque ladite unité de traitement (150) détermine qu'une période pendant laquelle la fluctuation de base déviant de ladite plage autorisée prédéterminée (221) a été détectée dépasse une période de temps prédéterminée, ladite unité de traitement (150) affiche un résultat de la détermination en plus du résultat de mesure de la forme d'onde électrocardiographique.

4. Électrocardiographe portable (100) selon la revendication 1, comprenant en outre un support de stockage détachable (132), dans lequel ladite unité de traitement (150) stocke dans ledit support de stockage externe (132) le résultat de mesure de la forme d'onde électrocardiographique conjointement avec un résultat de détection indiquant que la fluctuation de base dans la forme d'onde électrocardiographique dévie de la plage autorisée (221).

5. Méthode de traitement effectuée par un électrocardiographe portable ayant une électrode (120) mise en contact avec un corps vivant (250) et une unité d'affichage (148) affichant un résultat de mesure d'une forme d'onde électrocardiographique, comprenant une étape de traitement consistant à mesurer un signal électrique détecté par ladite électrode (120) en contact avec ledit corps vivant (250) sous la forme d'une forme d'onde électrocardiographique,
■ détecter que la fluctuation de base dans la forme d'onde électrocardiographique dévie d'une plage autorisée prédéterminée (221) (S5),
■ afficher sur ladite unité d'affichage (148) que la fluctuation de base déviant de ladite plage autorisée prédéterminée (221) a été détectée dans la forme d'onde électrocardiographique, sous la forme dudit résultat de mesure,
**caractérisée en ce que** ladite étape de traitement comprend l'étape consistant à afficher de façon distinguable la forme d'onde électrocardiographique pendant une période durant laquelle la fluctuation de base déviant de ladite plage autorisée prédéterminée (221) a été détectée, et à afficher également le résultat de mesure de la forme d'onde électrocardiographique sur l'ensemble d'une période de mesure.
